# FASCICULE DE BREVET EUROPEEN

(11) **EP 0 708 617 B1**
(45) Date de publication et mention de la délivrance du brevet: **08.12.1999**
(21) Numéro de dépôt: 94922910.8
(22) Date de dépôt: 12.07.1994
(51) Int. Cl.: A61B 17/00

(54) **SYSTEME D'OBTURATION TEMPORAIRE D'UN ORIFICE D'UN ORGANE PERFORE, TEL QU'EN PARTICULIER UN VAISSEAU**
VORRICHTUNG ZUR TEMPORÄREN ABDICHTUNG EINER ÖFFNUNG IN EINEM PERFORIERTEN ORGAN, INSBESONDERE EINEM GEFÄSS
SYSTEM FOR TEMPORARILY SEALING AN OPENING IN A PERFORATED ORGAN, ESPECIALLY A VESSEL

(30) Priorité: 21.07.1993 FR 9308950
(43) Date de publication de la demande: 01.05.1996
(73) Titulaire: Boussignac, Georges, 92160 Antony (FR)
(72) Inventeur: BOUSSIGNAC, Georges, F-92160 Antony (FR); HILAIRE, Pierre, F-75009 Paris (FR); PENDARIES, Pascal, F-75006 Paris (FR)
(74) Mandataire: Hubert, Philippe
(86) Numéro de dépôt international: FR9400872
(87) Numéro de publication internationale: WO9502997

(56) Documents cités:
- WO-A-90/04982
- DE-A- 3 203 410
- GB-A- 994 160
- US-A- 4 587 969
- US-A- 4 744 364
- US-A- 5 108 420

## Description

La présente invention a pour objet un système d'obturation temporaire d'un orifice d'un organe perforé, tel que en particulier un vaisseau ayant subi une intervention.

L'invention trouve application dans le domaine médical, principalement dans le traitement des affections des vaisseaux sanguins, mais peut être également utilisée dans le cadre du traitement d'autres organes creux tels que la vessie, la vésicule ou les intestins.

On sait que lors des interventions sur les vaisseaux, avec abord percutané, il est nécessaire d'empêcher toute hémorragie au moment du retrait de l'appareillage introduit dis le vaisseau.

Une première solution, couramment utilisée, pour y parvenir, consiste à appliquer une compression manuelle au point de ponction. Cependant, cette solution n'est pas entièrement satisfaisante, car elle nécessite soit l'intervention d'un spécialiste pendant une durée relativement longue (30 minutes à 2 heures), soit l'utilisation d'un système de compression assistée, encombrant, et généralement mal supporté par le patient.

Une autre solution, pour éviter les risques d'hémorragie, consiste à introduire sur le chemin de ponction, un petit tampon réalisé dans une matière biorésorbable comme par exemple du collagène. Si elle évite les inconvénients d'une compression manuelle, cette solution nécessite cependant l'implantation d'un matériau étranger, dont l'éventuelle migration dans le vaisseau peut s'avérer extrêmement dangereuse.

Dans ces conditions, la présente invention a pour but de résoudre le problème technique consistant en la fourniture d'un système d'obturation temporaire d'un orifice d'un organe perforé, d'une nouvelle conception, qui puisse être mis en oeuvre de façon relativement aisée, dans de bonnes conditions de sécurité pour le patient, et qui puisse être facilement réalisé à l'échelle industrielle.

La solution, conforme à la présente invention, pour résoudre ce problème technique consiste en un système d'obturation temporaire d'un orifice d'un organe perforé, tel que en particulier un vaisseau ayant subi une intervention, caractérisé en ce qu'il comprend :
- un élément obturateur déformable susceptible d'être introduit à l'état déformé à l'intérieur dudit organe au travers dudit orifice et constitué par une première portion de fil présentant en position d'utilisation une configuration sensiblement plane, ladite première portion de fil étant dévidable par traction à partir de son centre et comportant une face étanche destinée à être appliquée sur la face interne dudit organe, afin d'obturer ledit orifice ; et
- un élément extracteur constitué par une seconde portion de fil, prolongeant la première sensiblement en son centre et qui, en position d'utilisation, traverse ledit orifice et s'étend à l'extérieur dudit organe en formant ainsi un moyen permettant, par traction, de mettre en contact ladite face étanche avec la face interne de l'organe pour obturer ledit orifice pendant un temps suffisant pour permettre son rétrécissement par reconstruction naturelle de l'organe, puis d'extraire ladite portion de fil par dévidage, lorsque ledit orifice est suffisamment rétréci.

Comme on le comprend, ce nouveau système conforme à l'invention permet de réaliser une obturation mécanique interne et temporaire de l'organe perforé, pendant un temps suffisant pour permettre sa reconstitution naturelle.

Ce système est extrait lorsque l'orifice résultant de la perforation est suffisamment rétréci, de sorte que cette extraction est réalisée sans traumatiser la paroi du vaisseau.

Selon un mode de réalisation actuellement préféré de l'invention, la première portion de fil constituant l'élément obturateur déformable précité présente une configuration en spirale plane.

Selon un autre mode de réalisation de l'invention, elle présente une configuration en réseau tricoté.

Avantageusement, la première portion de fil précitée comme une face revêtue d'un film continu de polymère formant la face étanche précitée.

Selon une caractéristique particulière, l'élément obturateur précité est réalisé par chauffage sous pression d'un fil revêtu d'un polymère thermoplastique et formé en spirale par enroulement sur lui-même, ou tricoté.

Les première et seconde portions de fil peuvent être constituées de façon préférée par un fil unique, ou par des tronçons de fil reliés entre eux.

Selon une caractéristique particulière, les première et seconde portions de fil précitées peuvent être constituées de façon préférée par un micro-tube unique, ou par des tronçons de micro-tube reliés entre eux de façon étanche ; la partie de micro-tube constituant ladite première portion de fil comportant en outre dans la paroi la constituant des micro-trous. Cette conformation rend possible une injection intraparientale au travers de la première portion de fil d'un produit susceptible d'accélérer le phénomène de cicatrisation.

Selon une caractéristique particulière, le système d'obturation selon l'invention comprend :
- une première gaine à l'intérieur de laquelle est disposée une majeure partie de la seconde portion de fil constituant l'élément extracteur ;
- un poussoir comportant un logement longitudinal le traversant, de préférence de façon centrale, et destiné à loger ladite première gaine et dont l'extrémité est conformée pour permettre de pousser l'élément obturateur ;
- une deuxième gaine à l'intérieur de laquelle sont disposés ledit poussoir et ledit élément obturateur à l'état déformé.

L'invention sera mieux comprise et d'autres buts, caractéristiques et avantages de celle-ci apparaîtront plus clairement à la lecture de la description explicative qui va suivre, faite en référence aux dessins schématiques annexés donnés uniquement à titre d'exemple non limitatif illustrant un mode de réalisation actuellement préféré de l'invention et dans lesquels :
- les figures 1A à 1D illustrent un procédé de fabrication d'un élément obturateur et de l'élément extracteur associé selon un mode de réalisation actuellement préféré de l'invention et plus précisément :
   - la figure 1A illustre l'étape initiale de ce procédé ;
   - la figure 1B illustre l'enroulement en spirale de la première portion de fil constituant l'élément obturateur ;
   - la figure 1C illustre la réalisation de la face étanche de l'élément obturateur;
   - la figure 1D représente le produit issu du procédé résultant des étapes représentées aux figures 1A à 1C.
- la figure 2A est une vue en coupe longitudinale illustrant la mise en place de l'élément obturateur à l'état déformé à l'intérieur d'une gaine ;
- la figure 2B est une vue semblable à la figure 2A illustrant l'élément obturateur à l'intérieur de la gaine ;
- la figure 3A est une vue en coupe longitudinale illustrant la phase initiale d'introduction d'un système d'obturation conforme à la présente invention à l'intérieur d'un vaisseau ;
- la figure 3B est une vue semblable à la figure 3A illustrant l'opération de largage de l'élément obturateur à l'intérieur du vaisseau ;
- la figure 3C est une vue semblable aux précédentes, illustrant la mise en place de l'élément obturateur au contact de la face interne de l'organe afin d'obturer un orifice ;
- la figure 3D est une vue semblable aux précédentes illustrant le maintien en position d'utilisation de l'élément obturateur ;
- la figure 3E est une vue semblable aux précédentes illustrant la phase initiale de dévidage de la première portion de fil constituant l'élément obturateur ;
- la figure 3F est une vue semblable aux précédentes illustrant la phase finale de dévidage de la première portion de fil constituant l'élément obturateur ; et
- la figure 4 est une vue en coupe longitudinale d'un système d'obturation temporaire complet selon l'invention.

Dans la description qui va suivre, l'organe perforé choisi à titre d'exemple est un vaisseau sanguin 10 ayant subi une intervention, comme par exemple l'introduction d'un cathéter pour le traitement d'une sténose.

L'expression "position d'utilisation" utilisée dans le cadre de la présente description et des revendications désigne la position dans laquelle l'orifice 2 du vaisseau est obturé.

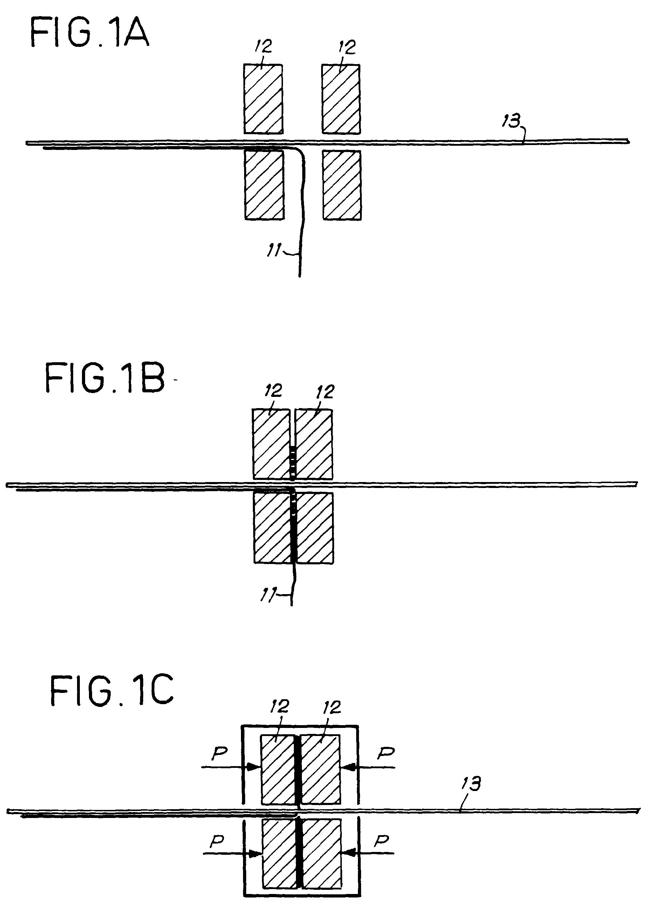

On décrira tout d'abord en référence aux figure 1A à 1D un procédé de fabrication d'un élément obturateur et de l'élément extracteur associé, selon un mode de réalisation actuellement préféré de l'invention.

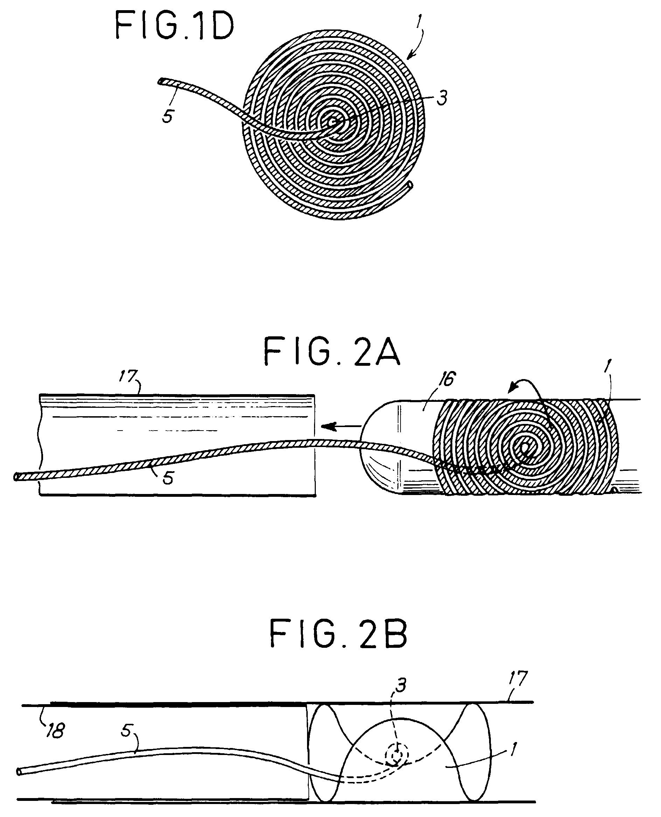

Selon ce mode de réalisation, l'élément obturateur présente sensiblement la forme d'une rondelle plate constituée par un fil ou tube 11 enroulé sur lui-même pour constituer une spirale, tandis que l'élément extracteur 5 est constitué par une portion de fil ou tube prolongeant ladite spirale sensiblement en son centre 3 (figure 1D).

Dans l'exemple représenté, les portions de fil constituant l'élément obturateur et l'élément extracteur sont constituées par un fil unique. Il est bien évident que ces éléments peuvent être également constitués par des tronçons de fil identiques ou différents reliés entre eux par tout moyen approprié.

En outre, les portions de fil constituant l'élément obturateur et l'élément extracteur peuvent être constituées par un micro-tube unique, ou par des des tronçons de micro-tube reliés entre eux de façon étanche ; la partie de micro-tube constituant l'élément obturateur comportant en outre dans sa paroi des micro-trous pour l'injection d'un produit susceptible d'accélérer la cicatrisation.

Le fil ou tube 11 utilisé sera avantageusement constitué d'une matière thermoplastique comme en particulier un polyamide, et de préférence un polyuréthane comme par exemple le produit commercialisé sous la dénomination Pellethane. Il peut être également constitué d'une armature métallique revêtue d'une matière thermoplastique telle que celle mentionnée précédemment.

Pour enrouler le fil 11 sur lui-même, on peut utiliser un moule constitué de deux cylindres 12 métalliques comportant chacun un trou sensiblement central destiné au passage d'une tige en Inox 13.

Dans une première étape représentée à la figure 1A, le fil 11 est passé au travers du trou de l'un des cylindres 12 de telle sorte que son extrémité libre soit disposée entre les deux cylindres 12.

Puis, les deux cylindres sont rapprochés l'un de l'autre et on enroule le fil 11 sur lui-même autour de la tige 13, entre lesdits cylindres, de façon à constituer ainsi une spirale comme représenté à la figure 1B.

Afin de constituer une face étanche 4 destinée à l'obturation de l'orifice de l'organe perforé, on dispose l'ensemble constitué du moule et du fil enroulé en spirale entre les deux cylindres, à l'intérieur d'une enceinte.

On applique ensuite une légère pression P sur le moule, puis on porte la température à l'intérieur de l'enceinte à une valeur suffisante pour réaliser une thermosoudure du fil sur lui-même (figure 1C).

Cette soudure doit être suffisante pour garantir une parfaite étanchéité de l'élément obturateur en position d'utilisation tout en permettant un dévidage de la portion de fil constituant la spirale sans détachement de particules de matières dans l'organisme.

Les valeurs de la pression P à appliquer sur le moule, de la température à l'intérieur de l'enceinte et du temps de chauffe pourront être facilement déterminées par l'homme de métier, pour parvenir au but recherché.

Dans l'exemple qui vient d'être décrit, l'élément obturateur présente une configuration en spirale plane.

Il est également envisageable de réaliser cet élément par tricotage d'un fil ou tube de même nature qu'indiquée précédemment, le réseau ainsi tricoté devant être dévidable à partir de son centre.

Une face étanche peut également être obtenue dans ce cas par chauffage sous pression de la pièce tricotée.

Les dimensions de l'élément obturateur 1 doivent bien entendu être supérieures à celles de l'orifice à obturer.

Cet élément obturateur étant destiné à être introduit à l'intérieur de l'organe perforé, il est donc nécessaire que sa structure soit déformable.

Cette possibilité sera généralement obtenue en réalisant l'élément obturateur sous forme plane, ce qui permet de le déformer par exemple par enroulement, de telle sorte que son encombrement à l'état déformé puisse permettre son introduction au travers de l'orifice de l'organe perforé.

D'une façon générale, l'élément obturateur 1 sera introduit à l'intérieur de l'organe au moyen d'un introducteur et d'un poussoir, comme il sera expliqué plus loin.

Pour faciliter son introduction à l'intérieur de l'élément introducteur, l'élément obturateur sera enroulé autour d'une matrice métallique 16 à tête hémisphérique, et introduit dans une gaine 17 dont les dimensions externes sont légèrement inférieures aux dimensons internes de l'élément introducteur. (Voir figure 2A).

Il est à noter que la portion de fil constituant l'élément extracteur 5 traverse la gaine 17 comme représenté.

Après avoir retiré la matrice cylindrique 16, on introduit à l'extrémité libre de la gaine un poussoir 18 susceptible d'être traversé par la portion de fil constituant l'élément extracteur 5.

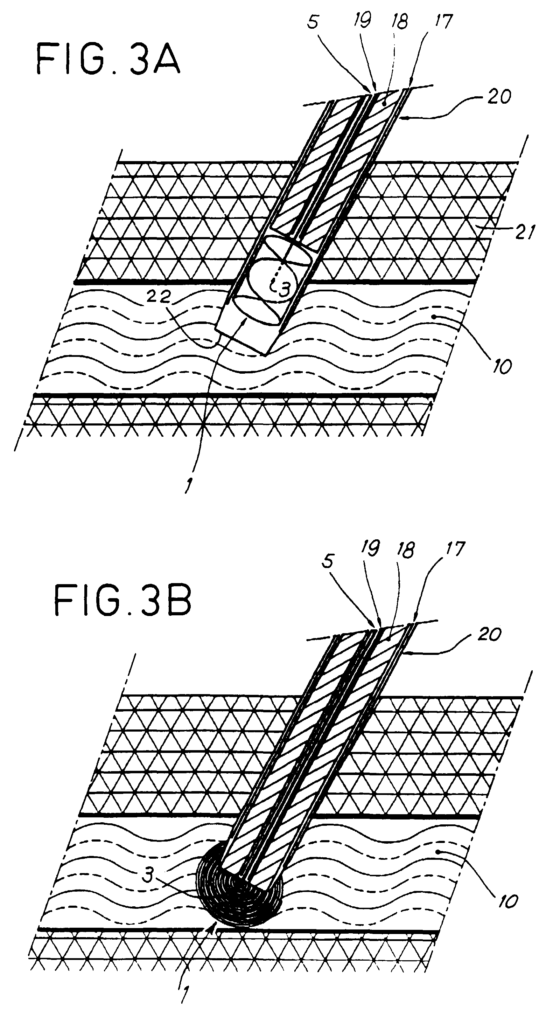

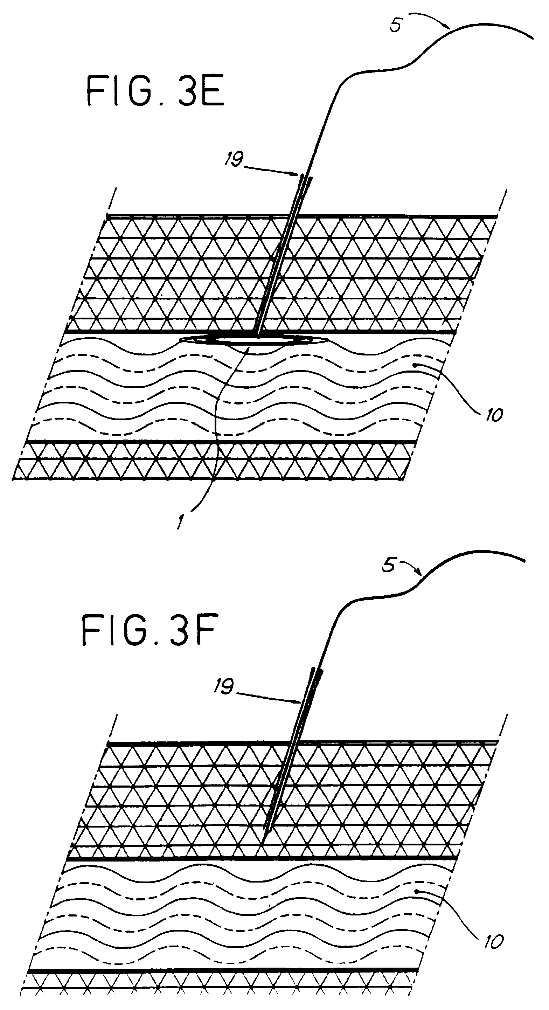

En référence aux figures 3A à 3F, on décrira maintenant la mise en oeuvre du système d'obturation temporaire conforme à l'invention.

La figure 3A est une vue en coupe longitudinale illustrant la phase initiale d'introduction de ce système à l'intérieur d'un vaisseau 10.

Le chiffre de référence 21 représente la structure extérieure à l'organe, c'est-à-dire, dans l'exemple choisi, l'ensemble épiderme-derme-graisse-muscle.

Le chiffre de référence 20 représente l'élément introducteur à l'intérieur duquel est disposé l'ensemble gaine 17-poussoir 18-élément obturateur 1 et élément extracteur 5.

Dans l'exemple représenté, la portion de fil constituant l'élément extracteur 5 est elle-même introduite à l'intérieur d'une gaine 19, pour faciliter le dévidage ultérieur.

En outre, le poussoir 18 comporte un logement longitudinal le traversant de façon sensiblement centrale destiné à recevoir cette gaine 19.

Lorsque l'extrémité 22 de l'introducteur 20 se trouve à l'intérieur du vaisseau 10, on procède alors au largage de l'élément obturateur 1 en déplaçant le poussoir 18 vers l'extrémité avant libre 22 de l'élément introducteur 20 (figure 3B).

L'élément obturateur 1 reprend alors sa forme initiale sensiblement plane.

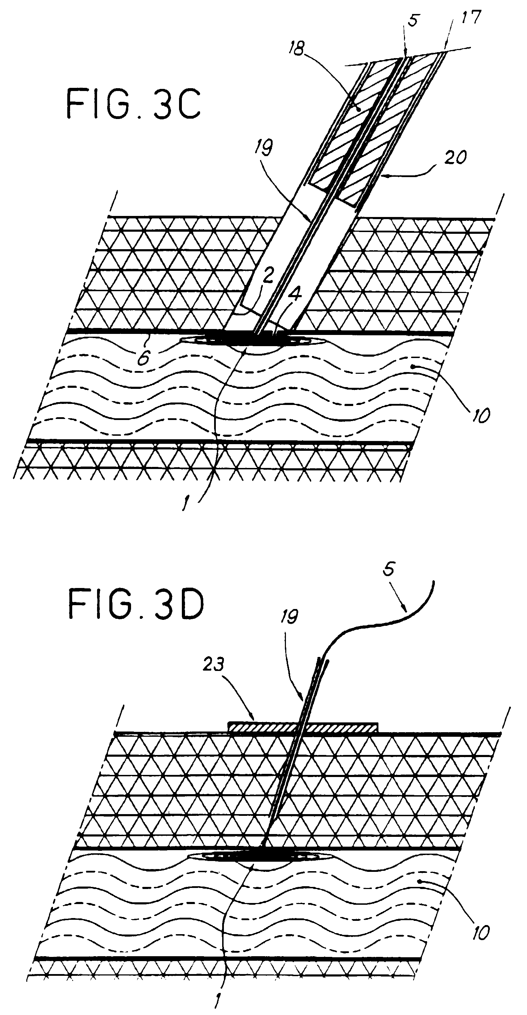

On retire ensuite l'élément introducteur 20, la gaine 17 et le poussoir 18 et on met la face étanche 4 de l'élément obturateur 1 en contact avec la face interne 6 de l'organe pour obturer l'orifice 2, par traction de la portion de fil constituant l'élément extracteur 5 (voir figure 3C).

L'extrémité avant de la gaine 19 à l'intérieur de laquelle se trouve la portion de fil définissant l'élément extracteur 5 est disposée au niveau de la surface de contact entre la face étanche 4 et la face interne 6.

Pour maintenir l'élément obturateur 1 en position d'obturation de l'orifice, il peut être avantageux d'immobiliser la gaine 19 à l'aide d'un élément de maintien tel qu'un clip 23, après avoir retiré de l'orifice 2 l'ensemble constitué par l'introducteur 20, la gaine 17 et le poussoir 18 (voir figure 3D).

L'élément obturateur 1 est maintenu dans cette position pendant un temps suffisant pour permettre le rétrécissement de l'orifice 2 par reconstruction naturelle de l'organe perforé.

Lorsque l'orifice est suffisamment rétréci, on dessère le clip 23 et, en maintenant l'ensemble clip 23-gaine 19, on tire la portion de fil constituant l'élément extracteur 5, de façon à dévider ainsi la portion de fil constituant l'élément obturateur 1 (figure 3E). On obtient finalement un orifice dont les dimensions sont celles de la gaine 19, c'est-à-dire des dimensions suffisamment petites pour éviter tout risque d'hémorragie, la cicatrisation de l'organe étant pratiquement complètement réalisée.

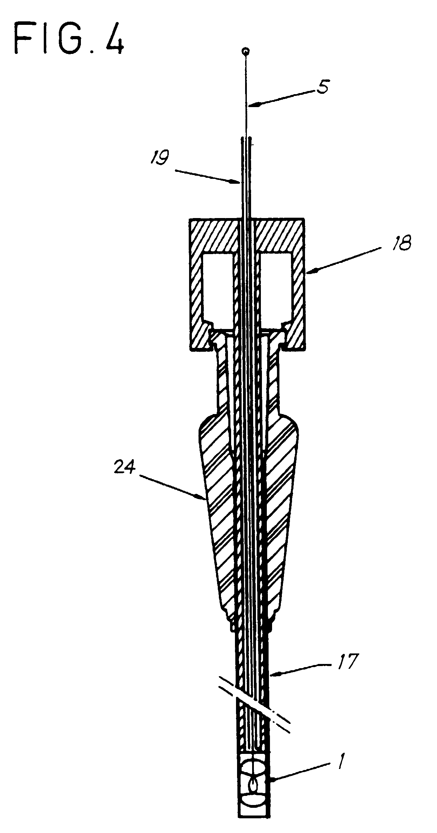

On a représenté à la figure 4 en coupe logitudinale un système d'obturation temporaire conforme à la présente invention.

Comme on peut le voir, ce système comporte en outre une embase 24 constituée par une bague susceptible d'être clipée sur la tête du poussoir 18 conformée à cet effet.

Cette embase est destinée à permettre de fixer le système d'introduction sur un introducteur par un effet de clipage.

Le système d'obturation qui vient d'être décrit est relativement aisé à mettre en oeuvre, dans de bonnes conditions de sécurité pour le patient et peut être réalisé relativement facilement à l'échelle industrielle.

## Revendications

1. Système d'obturation temporaire d'un orifice d'un organe perforé, tel que en particulier un vaisseau ayant subi une intervention, caractérisé en ce qu'il comprend :
- un élément obturateur déformable (1) susceptible d'être introduit à l'état déformé à l'intérieur dudit organe au travers dudit orifice (2) et constitué par une première portion de fil présentant en position d'utilisation une configuration sensiblement plane, ladite première portion de fil étant dévidable par traction à partir de son centre (3) et comportant une face étanche (4) destinée à être appliquée sur la face interne dudit organe, afin d'obturer ledit orifice (2) et
- un élément extracteur (5) constitué par une seconde portion de fil, prolongeant la première sensiblement en son centre (3) et qui, en position d'utilisation, traverse ledit orifice (2) et s'étend à l'extérieur dudit organe en formant ainsi un moyen permettant, par traction, de mettre en contact ladite face étanche (4) avec la face interne (6) de l'organe pour obturer ledit orifice (2) pendant un temps suffisant pour permettre son rétrécissement par reconstruction naturelle de l'organe, puis d'extraire ladite première portion de fil par dévidage, lorsque ledit orifice est suffisamment rétréci.

2. Système d'obturation selon la revendication 1, caractérisé en ce que la première portion de fil précitée présente une configuration en spirale plane.

3. Système d'obturation selon la revendication 1, caractérisé en ce que la première portion de fil précitée présente une configuration en réseau tricoté.

4. Système d'obturation selon la revendication 2 ou 3, caractérisé en ce que la première portion de fil précitée comporte une face revêtue d'un film continu de polymère formant la face étanche (4) précitée.

5. Système d'obturation selon la revendication 4, caractérisé en ce que l'élément obturateur (1) précité est réalisé par chauffage sous pression d'un fil revêtu d'un polymère thermoplastique et formé en spirale par enroulement sur luimême, ou tricoté.

6. Système d'obturation selon l'une des revendications 1 à 5, caractérisé en ce que les première et seconde portions de fil précitées sont constituées par un fil unique.

7. Système d'obturation selon la revendication 5, caractérisé en ce que le polymère thermoplastique précité est un polyuréthane.

8. Système d'obturation selon l'une des revendications 1 à 5, caractérisé en ce que les première et seconde portions de fil précitées peuvent être constituées de façon préférée pu un micro-tube unique, ou par des tronçons de micro-tube reliés entre eux de façon étanche; la partie de micro-tube constituant ladite première portion de fil comportant en outre dans la paroi la constituant des micro-trous.

9. Système d'obturation selon l'une quelconque des revendications 1 à 8, caractérisé en ce qu'il comprend :
- une première gaine (19) à l'intérieur de laquelle est disposée une majeure partie de la seconde portion de fil constituant l'élément extracteur (5);
- un poussoir (18) comportant un logement longitudinal le traversant, de préférence de façon centrale, et destiné à loger ladite première gaine et dont l'extrémité est conformée pour permettre de pousser l'élément obturateur ;
- une deuxième gaine (17) à l'intérieur de laquelle sont disposés ledit poussoir (18) et ledit élément obturateur (1) à l'état déformé.

10. Système d'obturation selon la revendication 9, caractérisé en ce qu'il comprend en outre un élément permettant de maintenir en position la première gaine (19), constitué par exemple d'un clip (23).

## Claims

1. A system for temporarily sealing an opening in a perforated organ, such as in particular a vessel having undergone an operation, characterised in that it comprises :
- a deformable sealing element (1) capable of being introduced in the deformed state inside said organ through said opening (2) and constituted by a first portion of thread having, in position of use, a substantially flat form, said first portion of thread being unwindable by traction from its centre (3) and comprising a liquid-fight face (4) intended to be applied on the inner face of said organ, in order to seal said opening (2) and
- an extractor element (5) constituted by a second portion of thread, extending the first substantially at its centre (3) and which, in position of use, passes through said opening (2) and extends outside said organ, thus forming a means making it possible, by traction, to bring said liquid-tight face (4) into contact with the inner face (6) of the organ in order to seal said opening (2) for a sufficient length of time to allow constriction thereof by natural reconstruction of the organ, then to extract said first portion of thread by unwinding, when said opening is sufficiently constricted.

2. The sealing system according to claim 1, characterised in that the above-mentioned first portion of thread has a flat spiral form.

3. The sealing system according to claim 1, characterised in that the above-mentioned first portion of thread has a knitted network form.

4. The sealing system according to claim 2 or 3, characterised in that the above-mentioned first portion of thread comprises a face coated with a continuous film of polymer forming the above-mentioned liquid-tight face (4).

5. The sealing system according to claim 4, characterised in that the above-mentioned sealing element (1) is made by heating, under pressure, a thread coated with a thermoplastic polymer and formed in a spiral by winding on itself or knitted.

6. The sealing system according to one of claims 1 to 5, characterised in that the above-mentioned first and second portions of thread are constituted by a single thread.

7. The sealing system according to claim 5, characterised in that the above-mentioned thermoplastic polymer is a polyurethane.

8. The sealing system according to one of claims 1 to 5, characterised in that the above-mentioned first and second portions of thread may preferably be constituted by a single micro-tube, or by sections of micro-tube connected together in liquid-tight manner ; the part of the micro-tube constituting said first portion of thread further comprising micro-holes in the wall constituting it.

9. The sealing system according to any one of claims 1 to 8, characterised in that it comprises :
- a first sheath (19) inside which is disposed a major part of the second portion of thread constituting the extractor element (5) ;
- a pusher (18) comprising a longitudinal housing traversing it, preferably centrally, and intended to house said first sheath and whose end is shaped to enable the sealing element to be pushed ;
- a second sheath (17) inside which are disposed said pusher (18) and said sealing element (1) in the deformed state.

10. The sealing system according to claim 9, characterised in that it further comprises an element enabling the first sheath (19) to be maintained in position, constituted for example by a clip (23).

## Patentansprüche

1. System zum vorübergehenden Verschließen einer Öffnung eines perforierten Organs, wie insbesondere eines einem Eingriff unterworfenen Gefäßes, dadurch gekennzeichnet, dass es
- ein verformbares Verschlusselement (1), das im verformten Zustand in das Innere des Organs durch die Öffnung (2) hindurch einführbar ist und das durch einen ersten Fadenabschnitt gebildet ist, der in der Gebrauchsstellung eine im Wesentlichen ebene Konfiguration aufweist, wobei der erste Fadenabschnitt durch Zug, ausgehend von seinem Zentrum (3), abwickelbar ist und eine dichte Seite (4) aufweist, die dazu vorgesehen ist, auf der Innenseite des Organs angebracht zu werden, um die Öffnung (2) zu verschließen, und
- ein Ziehelement (5) aufweist, das durch einen zweiten Fadenabschnitt gebildet ist, der den ersten im Wesentlichen in seinem Zentrum (3) verlängert und der sich in der Gebrauchsstellung durch die Öffnung (2) hindurch und zum Äußeren des Organs hinauserstreckt, wobei auf diese Weise ein Mittel gebildet ist, das erlaubt, die dichte Seite (4) mit der inneren Seite (6) des Organs durch Zug in Verbindung zu bringen, um die Öffnung (2) während einer Zeitdauer zu verschließen, die ausreicht, um deren Verengung durch einen natürlichen Wiederaufbau des Organs zu erlauben, und anschließend den ersten Fadenabschnitt durch Abwickeln herauszuziehen, wenn die Öffnung ausreichend verengt ist.

2. Verschließsystem nach Anspruch 1, dadurch gekennzeichnet, dass der erste Fadenabschnitt eine ebene Spiralenkonfiguration aufweist.

3. Verschließsystem nach Anspruch 1, dadurch gekennzeichnet, dass der erste Fadenabschnitt eine gestrickte Netzausbildung aufweist.

4. Verschließsystem nach Anspruch 2 oder 3, dadurch gekennzeichnet, dass der erste Fadenabschnitt eine Seite aufweist, die mit einem kontinuierlichen Polymerfilm überzogen ist, der die genannte dichte Seite (4) bildet.

5. Verschließsystem nach Anspruch 4, dadurch gekennzeichnet, dass das Verschlusselement (1) durch Erwärmung eines mit einem thermoplastischen Polymer überzogenen Fadens unter Druck gebildet ist und durch ein In-sich-selbst-Einrollen zu einer Spirale geformt oder gestrickt ist.

6. Verschließsystem nach einem der Ansprüche 1 bis 5, dadurch gekennzeichnet, dass der erste und zweite Fadenabschnitt durch einen einzigen Faden gebildet sind.

7. Verschließsystem nach Anspruch 5, dadurch gekennzeichnet, dass das thermoplastische Polymer ein Polyurethan ist.

8. Verschließsystem nach einem der Ansprüche 1 bis 5, dadurch gekennzeichnet, dass der erste und der zweite Fadenabschnitt vorzugsweise durch einen einzigen Mikroschlauch gebildet sein können oder durch Mikroschlauchstücke, die miteinander dicht verbunden sind; wobei der Teil des Mikroschlauchs, der den ersten Fadenabschnitt bildet, weiters in der diesen bildende Wand Mikrolöcher aufweist.

9. Verschließsystem nach einem der Ansprüche 1 bis 8, dadurch gekennzeichnet, dass sie:
- eine erste Hülle (19), in deren Innerem ein Großteil des zweiten Fadenabschnittes, der das Zugelement (5) bildet, vorgesehen ist;
- ein Vorschubelement (18), das ein durch diese vorzugsweise mittig durchgehendes Längslager aufweist, das dazu vorgesehen ist, die erste Hülle aufzunehmen, und dessen Ende so gebildet ist, um das Verschlusselement vortreiben zu können;
- eine zweite Hülle (17) aufweist, in deren Innerem das Vorschubelement (18) und das Verschlusselement (1) in verformtem Zustand vorgesehen sind.

10. Verschließsystem nach Anspruch 9, dadurch gekennzeichnet, dass sie weiters ein Element, das z.B. durch einen Clip (23) gebildet ist, aufweist, um die erste Hülle (19) in Position zu halten.
